(19) Europäisches Patentamt
European Patent Office
Office européen des brevets

(11) Veröffentlichungsnummer: **0 399 394 A1**

(12) **EUROPÄISCHE PATENTANMELDUNG**

(21) Anmeldenummer: **90109439.1**

(22) Anmeldetag: **18.05.90**

(51) Int. Cl.[5]: **C07D 401/06, A01N 43/42**

(30) Priorität: **26.05.89 DE 3917234**
**01.08.89 DE 3925422**

(43) Veröffentlichungstag der Anmeldung:
**28.11.90 Patentblatt 90/48**

(84) Benannte Vertragsstaaten:
**CH DE ES FR GB IT LI**

(71) Anmelder: **BASF Aktiengesellschaft**
**Carl-Bosch-Strasse 38**
**D-6700 Ludwigshafen(DE)**

(72) Erfinder: **Seele, Rainer, Dr.**
**Leiblstrasse 3**
**D-6701 Fussgoenheim(DE)**
Erfinder: **Brill, Gunter, Dr.**
**Am Schlossergraben 3**
**D-6733 Hassloch(DE)**
Erfinder: **Kober, Reiner, Dr.**
**Im Schlittweg 20**
**D-6701 Fussgoenheim(DE)**
Erfinder: **Saupe, Thomas, Dr.**
**Kressenwiesenweg 13**
**D-6902 Sandhausen(DE)**
Erfinder: **Westphalen, Karl-Otto, Dr.**
**Mausbergweg 58**
**D-6720 Speyer(DE)**
Erfinder: **Wuerzer, Bruno, Dr.**
**Ruedigerstrasse 13**
**D-6701 Otterstadt(DE)**

(54) **8-Azolylmethylchinoline.**

(57) 8-Azolylmethylchinoline I

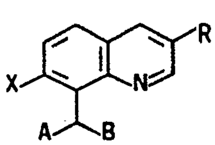

(I)

(R = H, Halogen, CN, $C_1$-$C_4$-Alkyl oder Halogenalkyl; X = Halogen; A = der 1-yl-Rest eines fünfgliedrigen, aromatischen Heterocyclus' mit 2 oder 3 H-Atomen im Ring, der noch weitere Substituenten tragen kann; B = Cl, Br, $C_1$-$C_4$-Alkoxy, $C_1$-$C_4$-Alkylthio, ein Aminrest, der mono- oder disubstituiert sein kann, ein Rest $ZR^4$ mit $R^4$ = Phenyl, das weitere Substituenten tragen kann, und Z = Sauerstoff, Schwefel oder ein Iminrest, der alkyliert sein kann) sowie die Säureadditionssalze und Metallkomplexe von I.
Die Verbindungen I und ihre Salze und Komplexe eignen sich als Herbizide.

EP 0 399 394 A1

# 8-Azolylmethylchinoline

Die vorliegende Erfindung betrifft neue 8-Azolylmethylchinoline der Formel I

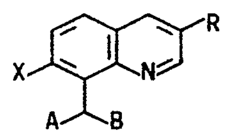

I

in der die Substituenten folgende Bedeutung haben:
R Wasserstoff, Halogen, eine Cyangruppe, eine $C_1$-$C_4$-Alkylgruppe, die mit bis zu 3 Halogenatomen substituiert sein kann;
X Halogen;
A eine 1,2-Diazol-1-yl-Gruppe, eine 1,3-Diazol-1-yl-Gruppe, eine 1,2,4-Triazol-1-yl-Gruppe oder eine 1,3,4-Triazol-1-yl-Gruppe, die an jedem der C-Atome eine der folgenden Substituenten tragen kann: Halogen, Thiol, Nitro, Cyan, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, $C_1$-$C_4$-Alkylsulfonyl, oder eine Gruppe CO-$R^1$, wobei $R^1$ $C_1$-$C_4$-Alkoxy oder eine Aminogruppe sein kann;
B Chlor, Brom, $C_1$-$C_4$-Alkoxy, $C_1$-$C_4$-Alkylthio, eine Gruppe -($NR^2R^3$), wobei $R^2$ und $R^3$ Wasserstoff, $C_1$-$C_4$-Alkyl oder $C_1$-$C_4$-Acyl bedeuten; eine Gruppe $ZR^4$, wobei $R^4$ Phenyl bedeutet, das eine Nitro-, Cyan- oder Phenoxygruppe und bis zu 3 der folgenden Substituenten tragen kann: Halogen, $C_1$-$C_4$-Alkyl, das bis zu 3 Halogenatome tragen kann, oder $C_1$-$C_4$-Alkoxy, und wobei Z Sauerstoff, Schwefel oder eine Gruppe -($NR^5$)-bedeutet, wobei $R^5$ Wasserstoff oder $C_1$-$C_4$-Alkyl ist,
sowie die Salze und Metallkomplexe von 1 mit solchen Säuren bzw. Metallen, welche die herbizide Wirkung von I nicht beeinträchtigen.

Außerdem betrifft die Erfindung Verfahren zur Herstellung dieser Verbindungen, ihre Verwendung als Herbizide sowie herbizide Mittel, welche diese Verbindungen als wirksame Substanzen enthalten.

Aus der DE-A 35 24 918 und der EP-B 60 429 sind 8-Azolylcarbonylchinoline bzw. 8-Carboxy- und 8-Dichlormethylchinoline als herbizid wirkende Verbindungen bekannt.

Die Selektivität dieser bekannten Herbizide bezüglich der Schadpflanzen vermag jedoch nur bedingt zu befriedigen, so daß der Erfindung neue herbizd wirksame Verbindungen als Aufgabe zugrunde lagen, mit denen sich die Schadpflanzen besser als bisher gezielt bekämpfen lassen, ohne dabei die Nutzpflanzen nennenswert anzugreifen.

Demgemäß wurden die eingangs definierten 8-Azolylmethylchinoline der Formel I gefunden.

Weiterhin wurden Verfahren zur Herstellung dieser 8-Azolylmethylchinoline gefunden, sowie die Verwendung dieser Verbindungen als Herbizide.

Die Verbindungen der Formel I enthalten ein asymmetrisches C-Atom und können somit als Enantiomere auftreten. Die Racemate lassen sich nach bekannten Methoden, beispielsweise durch Salzbildung mit einer optisch aktiven Säure, trennen. Als herbizide Mittel sind sowohl die reinen Enantiomeren, als auch das bei der Synthese anfallende Isomerengemisch geeignet.

Im einzelnen haben die Substituenten in den erfindungsgemäßen Verbindungen I folgende Bedeutung:
R vorzugsweise Wasserstoff, Halogen wie Fluor, Chlor, Brom, Jod, eine Cyangruppe, eine verzweigte oder unverzweigte $C_1$-$C_4$-Alkylgruppe, z. B. die Methyl-, Ethyl-, Propyl-, i-Propyl-, Trichlormethyl- und Trifluormethylgruppe, besonders bevorzugt Chlor und Brom;
X Halogen, besonders Fluor, Chlor und Brom;
A eine 1,2-Diazol-1-yl-Gruppe, eine 1,3-Diazol-1-yl-Gruppe, eine 1,2,4-Triazol-1-yl-Gruppe oder eine 1,3,4-Triazol-1-yl-Gruppe, die an jedem der C-Atome eine der folgenden Gruppen tragen kann: Halogen wie Fluor, Chlor und Brom, Thiol, Nitro, Cyano, $C_1$-$C_4$-Alkyl wie Methyl, partiell oder vollständig halogeniertes $C_1$-$C_4$-Alkyl, $C_1$-$C_4$-Alkoxy wie Methoxy, $C_1$-$C_4$-Alkylthio wie Methylthio, $C_1$-$C_4$-Alkylsulfonyl, $C_1$-$C_4$-Alkoxycarbonyl oder Aminocarbonyl; besonders Pyrazolyl, Imidazolyl, 2-Methylimidazolyl, 4-$COOCH_3$-imidazolyl, 4-$CONH_2$-imidazolyl, 5-Nitro-imidazolyl, 4,5-Dichlorimidazolyl, 1,2,4-Triazolyl, 1,3,4-Triazolyl, 5-Mercapto-1,2,4-Triazolyl, 5-Mercapto-1,3,4-Triazolyl;
B Chlor und Brom, eine verzweigte oder unverzweigte $C_1$-$C_4$-Alkoxygruppe, besonders Methoxy, Ethoxy und i-Propoxy, eine Phenoxygruppe, besonders Phenoxy, 2-Halogenphenoxy, 2-Fluorphenoxy, 4-Halogenphenoxy, 4-Chlor phenoxy, 2,4-Dihalogenphenoxy, 2,4-Dichlorphenoxy, 4-Trihalogenalkyl-phenoxy, 4-Trifluormethylphenoxy, 4-Alkoxy-phenoxy, 4-Methoxy-phenoxy und Phenoxyphenoxy, eine $C_1$-$C_4$-Alkylthio-

gruppe, besonders Methylthio und Ethylthio, eine Phenylthiogruppe, besonders Phenylthio und 4-Halogenphenylthio, 4-Chlorphenylthio, eine Aminogruppe, eine $C_1$-$C_4$-Alkylaminogruppe, besonders Methylamino und Ethylamino, eine Di-($C_1$-$C_4$)-Alkylaminogruppe wie Dimethylamino, eine $C_1$-$C_4$-Acylamino-oder N-($C_1$-$C_4$-Acyl)-N-($C_1$-$C_4$-Alkyl)-aminogruppe oder eine Phenylamino- oder Phenyl-alkylaminogruppe, besonders Phenylamino.

Geeignete Verbindungen sind in Tabelle I unter den Beispielen aufgeführt.

Besonders gut geeignet sind Verbindungen, bei denen R und X unabhängig voneinander Halogen, insbesondere Chlor, A eine 1,2,4-Triazol-1-yl- oder 1,3,4-Triazol-1-ylgruppe und B Chlor oder Brom bedeuten.

Als Säureadditionssalze eignen sich die Salze von solchen Säuren, welche die herbizide Wirkung von I nicht beeinträchtigen, also z.B. die Hydrochloride und -bromide, Sulfate, Nitrate, Phosphate, Oxalate oder die Dodecylbenzolsulfonate.

Als Metallkomplexe kommen die Komplexe des Kupfers, Zinks, Zinns, Mangans, Eisens, Kobalts oder Nickels in Betracht. Vorzugsweise stellt man die Komplexe aus den freien Basen I und mineralsauren Salzen, beispielsweise den Chloriden oder Sulfaten, der Metalle her.

Bedeutet B in den erfindungsgemäßen Verbindungen I Chlor oder Brom, so stellt man sie auf sehr vorteilhafte Weise analog der von H. Matsumoto et al (Tetrahedron Letters 52, 5011 (1979)) beschriebenen Methode durch Umsetzung der Azole AH mit einem 8-Formylchinolin II und einem anorganischen Säurehalogenid -($YHal_2$) entsprechend folgender Summengleichung her:

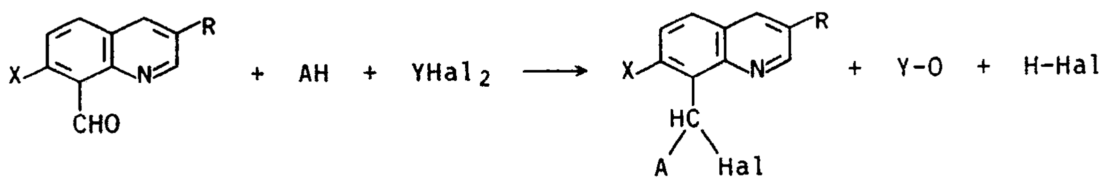

II Ia

Die anorganischen Säurehalogenide ($YHal_2$) sind Halogenierungsmittel wie Phosphoroxidchlorid, Thiophosgen, bevorzugt Phosgen, Thionylchlorid und -bromid.

Die Ausgangsverbindungen II sind bekannt oder in bekannter Weise erhältlich (vgl. EP-B-60 429), z.B. durch Oxidation substituierter 8-Methylchinoline der Formel III

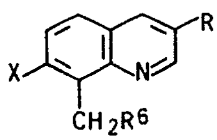

(III),

wobei $R^6$ Chlor, Brom, Methoxy oder Acetoxy bedeutet.

Als Oxidationsmittel können anorganische, oxidierende Säuren, Metalloxide wie Chromtrioxid oder Nitroalkane wie 2-Nitropropan verwendet werden.

Das Säurehalogenid wird bevorzugt in mindestens äquimolaren Mengen, insbesondere in der 1- bis 2fachen Menge, bezogen auf das Formylchinolin II eingesetzt. Die Azolkomponente A-H wird z.B. in der zweifachen, bevorzugt in der 5-6fachen molaren Menge, bezogen auf das Säurechlorid bzw. -bromid, eingesetzt.

Die Umsetzung erfolgt bevorzugt bei Temperaturen zwischen -30 und +100 °C, besonders bevorzugt zwischen 0 und 20 °C in Gegenwart eines Lösungsmittels.

Bevorzugte Lösungsmittel sind z.B. Nitrile wie Acetonitril, Ether wie Tetrahydrofuran, Diethylether oder Dioxan. Besonders bevorzugt werden Kohlenwasserstoffe und Chlorkohlenwasserstoffe wie Hexan, Benzol, Toluol, Methylenchlorid, Tetrachlorkohlenstoff oder Gemische der genannten Solventien.

Im allgemeinen arbeitet man bei Atmosphärendruck, sofern sich nicht wegen leichtflüchtiger Reaktionspartner ein höherer Druck, etwa bis zu 5 bar empfiehlt.

Da die Säurehalogenide und die intermediär entstehenden Zwischenverbindungen hydrolyseempfindlich sind, arbeitet man bevorzugt unter Feuchtigkeitsausschluß, besonders bevorzugt in einer Schutzgasatmosphäre.

Die Verbindungen I, in denen B einen anderen Substituenten als Chlor oder Brom bedeutet, lassen sich

besonders vorteilhaft aus den Chlor- oder Bromverbindungen Ia herstellen, indem man diese mit einer Verbindung H-B sowie einer Base umsetzt.

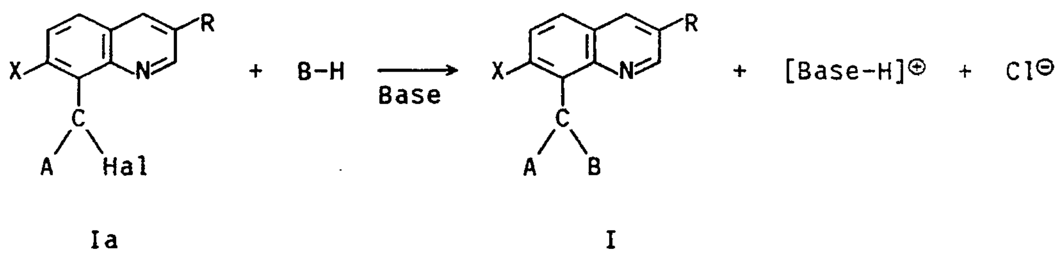

Die Komponente B-H wird vorteilhaft in stöchiometrischen Mengen, bevorzugt in ca. 20 %igem Überschuß eingesetzt, bezogen auf die 8-Azolylhalogenmethylchinoline Ia.

Die Reaktion erfolgt vorteilhaft unter Zusatz einer organischen oder anorganischen Hilfsbase und/oder eines Reaktionsbeschleunigers in Gegenwart eines Lösungsmittels.

Die Menge an Base und Reaktionsbeschleuniger kann je nach der eingesetzten Verbindung variiert werden. Vorteilhaft setzt man einen geringen Überschuß an Base, bezogen auf das 8-Azolylchinolin Ia, ein.

Geeignete Basen sind beispielsweise Alkalihydroxide wie Lithium-, Natrium- oder Kaliumhydroxid, Alkalicarbonate oder -hydrogencarbonate wie Natrium- oder Kaliumcarbonat oder Natrium- oder Kaliumhydrogencarbonat, Alkaliamide wie Natrium- oder Kaliumamid sowie die organischen Basen Pyridin, 4-Dialkylaminopyridin, Dialkylamine und Dialkylaniline oder bevorzugt das Alkalisalz der Komponente B-H.

Es kann auch die zur Herstellung der 8-Azolylmethylchinoline Ia eingesetzte Reaktionskomponente A-H als Base verwendet werden.

Der Reaktionsbeschleuniger wird bevorzugt in katalytischen Mengen zum Reaktionsgemisch gegeben.

Als Reaktionsbeschleuniger können z.B. Metallhalogenide, bevorzugt Natriumjodid oder Kaliumjodid, quartäre Ammoniumsalze wie Tetraalkylammoniumhalogenide oder -hydrogensulfate, bevorzugt Tetrabutylammoniumchlorid, -bromid oder -jodid, Aryltrialkylammoniumhalogenide wie Benzyltriethylammoniumchlorid oder -bromid und Kronenether wie 12-Krone-4, 15-Krone-5, Benzo-15-Krone-5, Dibenzo-18-Krone-6 oder Dicyclohexano-18-Krone-6, verwendet werden.

Als Lösungsmittel werden bevorzugt Ketone wie Aceton, Methylethylketon oder Cyclohexanon, Nitrile wie Acetonitril oder Propionitril, Alkohole wie Methanol, Ethanol, i-Propanol, n-Butanol, Glykole, Ester wie Essigsäuremethylester, Essigsäureethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder Gemische der genannten Lösungsmittel verwendet.

Die Umsetzung wird vorteilhaft bei Temperaturen zwischen 0 und 180° C, bevorzugt bei der Siedetemperatur des verwendeten Lösungsmittels, durchgeführt.

Bezüglich des Druckes gelten die Angaben für die Herstellung der Verbindung Ia.

Die erfindungsgemäßen Verfahren zur Herstellung substituierter 8-Azolylmethylchinoline können kontinuierlich oder diskontinuierlich durchgeführt werden.

Die Verbindungen I und ihre definitionsgemäßen Salze und Komplexe eignen sich als Herbizide.

## Herstellungsbeispiele

### Beispiel 1

3,7-Dichlor-8-[(1,2,4-triazol-1-yl)-chlormethyl]-chinolin

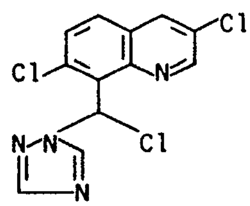

Eine Lösung aus 29,7 g (0,43 mol) Triazol in 150 ml Methylenchlorid wurde bei 0 °C unter Stickstoffatmosphäre mit 12,8 g (0,11 mol) Thionylchlorid und nach 30 minütigem Rühren bei 25 °C mit 16,3 g ($7,2 \cdot 10^{-2}$ mol) 3,7-Dichlor-chinolin-8-carbaldehyd versetzt.

Nach 12-stündiger Reaktionszeit bei 25 °C wurden 100 ml Wasser zugegeben, wonach die abgetrennte wäßrige Phase zweimal mit Methylenchlorid extrahiert wurde. Die vereinigten organischen Phasen wurden sodann wie üblich auf das 8-Azolylchlormethyl-chinolinderivat aufgearbeitet. Nach Umkristallisation aus Isopropanol wurden 19 g Produkt erhalten.
Ausbeute: 84 %; Fp.: 140 - 146 °C

Beispiel 2

3,7-Dichlor-8-[(1,2,4-triazol-1-yl)-methoxymethyl]-chinolin

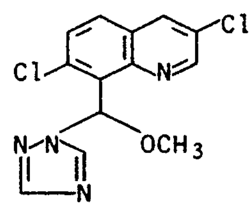

Eine Lösung aus 5 g ($1,6 \cdot 10^{-2}$ mol) 3,7-Dichlor-8-[(1,2,4-triazol-1-yl)-chlormethyl]-chinolin (Beispiel 1) in 100 ml Methanol, 2,2 g ($4,1 \cdot 10^{-2}$ mol) Natriummethylat und einer Spatelspitze Kaliumjodid wurde fünf Stunden unter Rückfluß erhitzt und anschließend mit 100 ml Wasser versetzt. Nach Extraktion mit Methyl-tert.-butylether wurde die organische Phase gewaschen und wie üblich aufgearbeitet.
Ausbeute: 4,5 g (91 %); Fp.: 156 - 158 °C

Analog den Beispielen 1 und 2 wurden die in Tabelle 1 aufgeführten Verbindungen hergestellt.

Tabelle 1

Neue herbizide 8-Azolylmethylchinoline

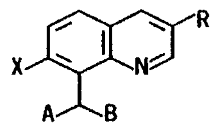

| Bsp. | R | X | A | B | Fp. [°C] | Herst. analog Bsp. |
|---|---|---|---|---|---|---|
| 3 | Cl | Cl | 1,2,4-Triazolyl | Br | 155 | 1 |
| 4 | Cl | Cl | 1,2,4-Triazolyl | $OC_2H_5$ | | 2 |
| 5 | Cl | Cl | 1,2,4-Triazolyl | O-iso-$C_3H_7$ | 119-125 | 2 |
| 6 | Cl | Cl | 1,2,4-Triazolyl | $SCH_3$ | | 2 |
| 7 | Cl | Cl | 1,2,4-Triazolyl | $NHCH_3$ | | 2 |
| 8 | Cl | Cl | 1,2,4-Triazolyl | S-$C_6H_5$ | | 2 |
| 9 | Cl | Cl | 1,2,4-Triazolyl | NH-$C_6H_5$ | | 2 |
| 10 | Cl | Cl | 1,2,4-Triazolyl | O-$C_6H_5$ | | 2 |
| 11 | Cl | Cl | 1,2,4-Triazolyl | 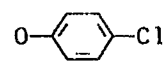 | | 2 |
| 12 | Cl | Cl | 1,2,4-Triazolyl | 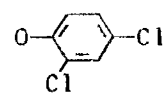 | | 2 |
| 13 | Cl | Cl | 1,2,4-Triazolyl | 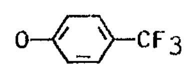 | | 2 |

Tabelle 1 (Fortsetzung)

| Bsp. | R | X | A | B | Fp. [°C] | Herst. analog Bsp. |
|---|---|---|---|---|---|---|
| 14 | Cl | Cl | Pyrazolyl | Cl | | 1 |
| 15 | Cl | Cl | Pyrazolyl | Br | | 1 |
| 16 | Cl | Cl | Pyrazolyl | $OCH_3$ | | 2 |
| 17 | Cl | Cl | Imidazolyl | Cl | | 1 |
| 18 | Cl | Cl | Imidazolyl | $OCH_3$ | | 2 |
| 19 | Cl | Cl | 5-Nitro-imidazolyl | Cl | | 1 |
| 20 | Cl | Cl | 5-Nitro-imidazolyl | $OC_2H_5$ | | 2 |
| 21 | Cl | Cl | 4,5-Dichlorimidazolyl | Cl | Harz | 1 |
| 22 | Cl | Cl | 4,5-Dichlorimidazolyl | $OCH_3$ | | 2 |
| 23 | Cl | Cl | 2-Methylimidazolyl | Cl | | 1 |
| 24 | Cl | Cl | 4-Cyano-imidazolyl | Cl | Harz | 1 |
| 25 | Cl | Cl | 4-Cyano-imidazolyl | $OCH_3$ | | 2 |
| 26 | Cl | Cl | 4-Cyano-imidazolyl | $SCH_3$ | | 2 |
| 27 | Cl | Cl | 4-$CONH_2$-imidazolyl | Cl | 174-180 | 1 |
| 28 | Cl | Cl | 4-$CONH_2$-imidazolyl | $OCH_3$ | | 2 |
| 29 | Cl | Cl | 4-$CONH_2$-imidazolyl | $SCH_3$ | | 2 |
| 30 | Cl | Cl | 4-$CONH_2$-imidazolyl | O-$C_6H_5$ | | 2 |
| 31 | Cl | Cl | 4-$CONH_2$-imidazolyl | NH-$C_6H_5$ | | 2 |
| 32 | Cl | Cl | 4-$CONH_2$-imidazolyl | S-$C_6H_5$ | | 2 |
| 33 | Cl | Cl | 4-$COOCH_3$-imidazolyl | Cl | Harz | 1 |
| 34 | Cl | Cl | 4-$COOCH_3$-imidazolyl | $OCH_3$ | | 2 |
| 35 | Cl | Cl | 4-$COOCH_3$-imidazolyl | $OC_2H_5$ | | 2 |
| 36 | Cl | Cl | 4-$COOCH_3$-imidazolyl | O-$C_6H_5$ | | 2 |

Tabelle 1 (Fortsetzung)

| Bsp. | R | X | A | B | Fp. [°C] | Herst. analog Bsp. |
|---|---|---|---|---|---|---|
| 37 | Cl | Cl | 4-$COOCH_3$-imidazolyl | S-$C_6H_5$ | | 2 |
| 38 | Cl | Cl | 5-Mercapto-1,2,4-triazolyl | Cl | | 1 |
| 39 | Cl | Cl | 5-Mercapto-1,2,4-triazolyl | $OCH_3$ | | 2 |
| 40 | F | Cl | 1,2,4-Triazolyl | Cl | | 1 |
| 41 | F | Cl | 1,2,4-Triazolyl | $OCH_3$ | | 2 |
| 42 | F | Cl | 1,2,4-Triazolyl | Br | | 1 |
| 43 | Br | Cl | 1,2,4-Triazolyl | Cl | | 1 |
| 44 | Cl | F | 1,2,4-Triazolyl | Cl | | 1 |
| 45 | Cl | F | 1,2,4-Triazolyl | $OCH_3$ | | 2 |
| 46 | Cl | Br | 1,2,4-Triazolyl | Cl | | 1 |
| 47 | $CH_3$ | Cl | 1,2,4-Triazolyl | Cl | 148-155 | 1 |
| 48 | $CH_3$ | Cl | 1,2,4-Triazolyl | $OCH_3$ | 151-154 | 2 |
| 49 | $CH_3$ | Cl | 1,2,4-Triazolyl | Br | | 1 |
| 50 | $CH_3$ | Cl | 1,2,4-Triazolyl | $OC_2H_5$ | | 2 |
| 51 | $CH_3$ | Cl | 1,2,4-Triazolyl | $SCH_3$ | | 2 |
| 52 | $CH_3$ | Cl | 1,2,4-Triazolyl | O-$C_6H_5$ | | 2 |
| 53 | $CH_3$ | Cl | 1,2,4-Triazolyl | S-$C_6H_5$ | | 2 |
| 54 | $CH_3$ | Cl | 1,2,4-Triazolyl | NH-$C_6H_5$ | | 2 |
| 55 | $CH_3$ | Cl | 1,2,4-Triazolyl | 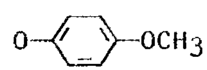 | | 2 |
| 56 | $CH_3$ | Cl | 1,2,4-Triazolyl | 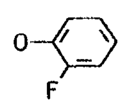 | | 2 |

Tabelle 1 (Fortsetzung)

| Bsp. | R | X | A | B | Fp. [°C] | Herst. analog Bsp. |
|---|---|---|---|---|---|---|
| 57 | $CH_3$ | Cl | Imidazolyl | Cl | | 1 |
| 58 | $CH_3$ | Cl | Imidazolyl | $OCH_3$ | | 2 |
| 59 | $CH_3$ | Cl | Pyrazolyl | Cl | | 1 |
| 60 | $CH_3$ | Cl | Pyrazolyl | Br | | 1 |
| 61 | $CH_3$ | Cl | Pyrazolyl | $OCH_3$ | | 2 |
| 62 | $CH_3$ | Cl | Pyrazolyl | $O-C_6H_5$ | | 2 |
| 63 | $CH_3$ | Cl | Pyrazolyl | 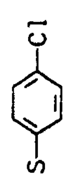 | | 2 |
| 64 | $CH_3$ | Cl | 4-$CONH_2$-imidazolyl | Cl | | 1 |
| 65 | $CH_3$ | Cl | 4-$CONH_2$-imidazolyl | $OCH_3$ | | 2 |
| 66 | H | Cl | 1,2,4-Triazolyl | Cl | | 1 |
| 67 | H | Cl | 1,2,4-Triazolyl | $OCH_3$ | | 2 |
| 68 | $CF_3$ | Cl | 1,2,4-Triazolyl | Cl | | 1 |
| 69 | $CF_3$ | Cl | Imidazolyl | Cl | | 1 |
| 70 | $CF_3$ | Cl | Imidazolyl | $OCH_3$ | | 2 |
| 71 | $CF_3$ | Cl | Imidazolyl | $OC_6H_5$ | | 2 |
| 72 | CN | Cl | 1,2,4-Triazolyl | Cl | | 1 |
| 73 | CN | Cl | 1,2,4-Triazolyl | $OCH_3$ | | 2 |
| 74 | CN | Cl | Pyrazolyl | Cl | | 1 |
| 75 | CN | Cl | Pyrazolyl | $OCH_3$ | | 2 |

Die 8-Azolylmethylchinoline bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid (?-USA), Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 bis 100 % (nach NMR-Spektrum) eingesetzt.

Beispiele für solche Zubereitungen sind:

1. eine Lösung aus 90 Gew.-Teilen der Verbindung Nr. 1 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 2, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine Dispersion, die 0,02 Gew.-% des Wirkstoffs enthält.

III. eine wäßrige Dispersion aus 20 Gew.-Teilen der Verbindung Nr. 3, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.

IV. eine wäßrige Dispersion aus 20 Gew.-Teilen der Verbindung Nr. 5, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;

V. eine in einer Hammermühle vermahlene Mischung aus 80 Gew.-Teilen der Verbindung Nr. 21, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;

VI. eine innige Mischung aus 3 Gew.-Teilen der Verbindung Nr. 24 und 97 Gew.-Teilen feinteiligem Kaolin. Dieses Stäubemittel enthält 3 Gew.-% Wirkstoff;

VII. eine innige Mischung aus 30 Gew.-Teilen der Verbindung Nr. 27 92 Gew.-Teilen pulverförmigem Kieselsäuregel und 8 Gew.-Teilen Paraffinöl, das auf die Oberfläche dieses Kieselsäuregels gesprüht

wurde. Diese Aufbereitung gibt dem Wirkstoff eine gute Haftfähigkeit;

VIII. eine stabile wäßrige Dispersion aus 40 Gew.-Teilen der Verbindung Nr. 33, 10 Gew.-Teilen des Natriumsalzes eines Phenosulfonsäureharnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 47, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls;

X. eine in einer Hammermühle vermahlene Mischung aus 10 Gew.-Teilen der Verbindung Nr. 48, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 2,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |

| Botanischer Name | Deutscher Name |
|---|---|
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |

| Botanischer Name | Deutscher Name |
|---|---|
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die 8-Azolylmethylchinoline I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die herbiziden Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Anwendungsbeispiele

Die herbizide Wirkung der 8-Azolylmethylchinoline der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt.

Die Aufwandmenge betrug für Vor- und Nachauflaufbehandlung jeweils 1,0 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Botanischer Name | Deutscher Name |
|---|---|
| Cassia tora | Cassia |
| Galium aparine | Klettenlabkraut |
| Solanum nigrum | Schwarzer Nachtschatten |
| Zea mays | Mais |

Im Vorauflauf- und Nachauflaufverfahren ließen sich mit den Verbindungen 1 und 3 die breitblättrigen Pflanzen sehr gut bekämpfen, bei gleichzeitiger guter Verträglichkeit für die Kultur Mais (im Vorauflaufverfahren getestet).

## Ansprüche

1. Substituierte 8-Azolylmethylchinoline der allgemeinen Formel I

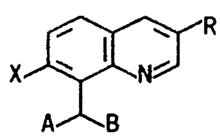

I

in der die Substituenten folgende Bedeutung haben:
R Wasserstoff, Halogen, eine Cyangruppe, eine $C_1$-$C_4$-Alkylgruppe, die mit bis zu 3 Halogenatomen substituiert sein kann;
X Halogen;
A eine 1,2-Diazol-1-yl-Gruppe, eine 1,3-Diazol-1-yl-Gruppe, eine 1,2,4-Triazol-1-yl-Gruppe oder eine 1,3,4-Triazol-1-yl-Gruppe, die an jedem der C-Atome eine der folgenden Substituenten tragen kann: Halogen, Thiol, Nitro, Cyan, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, $C_1$-$C_4$-Alkylsulfonyl, oder eine Gruppe CO-$R^1$, wobei $R^1$ $C_1$-$C_4$-Alkoxy oder eine Aminogruppe sein kann;
B Chlor, Brom, $C_1$-$C_4$-Alkoxy, $C_1$-$C_4$-Alkylthio, eine Gruppe -($NR^2R^3$), wobei $R^2$ und $R^3$ Wasserstoff, $C_1$-$C_4$-Alkyl oder $C_1$-$C_4$-Acyl bedeuten; eine Gruppe $ZR^4$, wobei $R^4$ Phenyl bedeutet, das eine Nitro-, Cyan- oder Phenoxygruppe und bis zu 3 der folgenden Substituenten tragen kann: Halogen, $C_1$-$C_4$-Alkyl, das bis zu 3 Halogenatome tragen kann, oder $C_1$-$C_4$-Alkoxy, und wobei Z Sauerstoff, Schwefel oder eine Gruppe -($NR^5$)- bedeutet, wobei $R^5$ Wasserstoff oder $C_1$-$C_4$-Alkyl ist,
sowie die Salze und Metallkomplexe von I mit solchen Säuren bzw. Metallen, welche die herbizide Wirkung von I nicht beeinträchtigen.

2. Verfahren zur Herstellung der substituierten 8-Azolylmethylchinoline der Formel I gemäß Anspruch 1, in der B Chlor oder Brom bedeutet, dadurch gekennzeichnet, daß man ein 8-Formylchinolin der Formel II

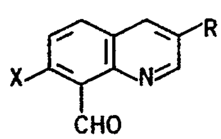

II

mit einem Azol der Formel A-H in Gegenwart von anorganischen Säurechloriden oder -bromiden umsetzt und die erhaltenen Verbindungen gewünschtenfalls in ihre Salze oder Metallkomplexe überführt.

3. Verfahren zur Herstellung der substituierten 8-Azolylmethylchinoline der Formel I gemäß Anspruch 1, in der B für einen anderen Substituenten als Chlor oder Brom steht, dadurch gekennzeichnet, daß man ein 8-Azolylmethylchinolin der Formel Ia

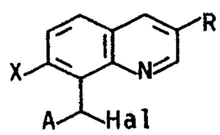

Ia

in der Hal Chlor oder Brom bedeutet, mit einer Verbindung der Formel B-H und einer Base umsetzt und die erhaltenen Verbindungen gewünschtenfalls in ihre Salze oder Metallkomplexe überführt.

4. Verwendung der 8-Azolylmethylchinoline I, deren Säureadditionssalzen und Metallkomplexen gemäß Anspruch 1 als Herbizide.

5. Herbizide Mittel, enthaltend ein 8-Azolylmethylchinolin der Formel I, dessen Säureadditionssalze oder Metallkomplexe gemäß Anspruch 1 und einen flüssigen oder festen Trägerstoff.

6. Verfahren zur Bekämpfung unerwünschten Pflanzenwuchses, dadurch gekennzeichnet, daß man eine herbizid wirksame Menge eines substituierten 8-Azolylmethylchinolins der Formel I, von dessen Säureadditionssalzen oder Metallkomplexen gemäß Anspruch 1 auf Pflanzen, deren Lebensraum oder auf Saatgut einwirken läßt.

7. Substituierte 8-Azolylmethylchinoline der Formel I nach Anspruch 1, wobei R und X Halogen, A eine Triazol-1-ylgruppe und B Chlor oder Brom bedeuten.

Patentansprüche für folgenden Vertragsstaat: ES

1. Verfahren zur Herstellung substituierter 8-Azolylmethylchinoline der Formel I

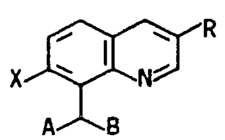

I

in der die Substituenten folgende Bedeutung haben:
R Wasserstoff, Halogen, eine Cyangruppe, eine $C_1$-$C_4$-Alkylgruppe, die mit bis zu 3 Halogenatomen substituiert sein kann;
X Halogen;
A eine 1,2-Diazol-1-yl-Gruppe, eine 1,3-Diazol-1-yl-Gruppe, eine 1,2,4-Triazol-1-yl-Gruppe oder eine 1,3,4-Triazol-1-yl-Gruppe, die an jedem der C-Atome eine der folgenden Substituenten tragen kann: Halogen, Thiol, Nitro, Cyan, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, $C_1$-$C_4$-Alkylsulfonyl, oder eine Gruppe CO-$R^1$, wobei $R^1$ $C_1$-$C_4$-Alkoxy oder eine Aminogruppe sein kann;
B Chlor oder Brom
sowie die Salze und Metallkomplexe von I mit solchen Säuren bzw. Metallen, welche die herbizide Wirkung von I nicht beeinträchtigen, dadurch gekennzeichnet, daß man ein 8-Formylchinolin der Formel II

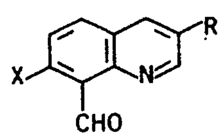

II

mit einem Azol der Formel A-H in Gegenwart von anorganischen Säurechloriden oder -bromiden umsetzt und die erhaltenen Verbindungen gewünschtenfalls in ihre Salze oder Metallkomplexe überführt.

2. Verfahren zur Herstellung substituierter 8-Azolylmethylchinoline der Formel I

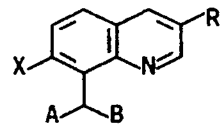

I

in der die Substituenten folgende Bedeutung haben:
R Wasserstoff, Halogen, eine Cyangruppe, eine $C_1$-$C_4$-Alkylgruppe, die mit bis zu 3 Halogenatomen substituiert sein kann;
X Halogen;
A eine 1,2-Diazol-1-yl-Gruppe, eine 1,3-Diazol-1-yl-Gruppe, eine 1,2,4-Triazol-1-yl-Gruppe oder eine 1,3,4-Triazol-1-yl-Gruppe, die an jedem der C-Atome eine der folgenden Substituenten tragen kann: Halogen, Thiol, Nitro, Cyan, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, $C_1$-$C_4$-Alkylsulfonyl, oder eine Gruppe CO-$R^1$, wobei $R^1$ $C_1$-$C_4$-Alkoxy oder eine Aminogruppe sein kann;
B $C_1$-$C_4$-Alkoxy, $C_1$-$C_4$-Alkylthio, eine Gruppe -($NR^2R^3$), wobei $R^2$ und $R^3$ Wasserstoff, $C_1$-$C_4$-Alkyl oder $C_1$-$C_4$-Acyl bedeuten; eine Gruppe $ZR^4$, wobei $R^4$ Phenyl bedeutet, das eine Nitro-, Cyan- oder Phenoxygruppe und bis zu 3 der folgenden Substituenten tragen kann: Halogen, $C_1$-$C_4$-Alkyl, das bis zu 3 Halogenatome tragen kann, oder $C_1$-$C_4$-Alkoxy, und wobei Z Sauerstoff, Schwefel oder eine Gruppe -($NR^5$)- bedeutet, wobei $R^5$ Wasserstoff oder $C_1$-$C_4$-Alkyl ist,
sowie die Salze und Metallkomplexe von I mit solchen Säuren bzw. Metallen, welche die herbizide Wirkung von I nicht beeinträchtigen, dadurch gekennzeichnet, daß man ein 8-Azolylmethylchinolin der Formel Ia

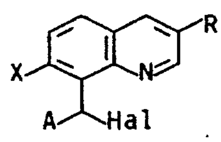

Ia

in der Hal Chlor oder Brom bedeutet, mit einer Verbindung der Formel B-H und einer Base umsetzt und die erhaltenen Verbindungen gewünschtenfalls in ihre Salze oder Metallkomplexe überführt.

3. Herbizide Mittel, enthaltend ein 8-Azolylmethylchinolin der Formel I, dessen Säureadditionssalz oder Metallkomplex gemäß Anspruch 1 oder 2, und einen flüssigen oder festen Trägerstoff.

4. Verfahren zur Bekämpfung unerwünschten Pflanzenwuchses, dadurch gekennzeichnet, daß man eine herbizid wirksame Menge eines substituierten 8-Azolylmethylchinolins der Formel I, von dessen Säureadditionssalzen oder Metallkomplexen gemäß Anspruch 1 oder 2, auf Pflanzen, deren Lebensraum oder auf Saatgut einwirken läßt.

Europäisches Patentamt

# EUROPÄISCHER RECHERCHENBERICHT

Nummer der Anmeldung

EP 90109439.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| D,A | DE - A1 - 3 108 873 (BASF) * Beispiele 40,52,98; Ansprüche 1,3,4 * -- | 1,5 | C 07 D 401/06<br>A 01 N 43/42 |
| A | DE - A1 - 2 648 826 (JCJ) * Anspruch 1; Beispiel 11 * -- | 1 | |
| D,A | DE - A1 - 3 524 918 (BASF) * Ansprüche 1,3 * -- | 1,5 | |
| A | CHEMICAL ABSTRACTS, Band 89, Nr. 3, 17. Juli 1978, Columbus, Ohio, USA MILLER, ALFRED DAVID et al. "Triazole derivatives" Seite 658, Spalte 1, Zusammenfassung-Nr. 24 368q & S. African 76 06,188 ---- | 1 | |
| | | | RECHERCHIERTE SACHGEBIETE (Int Cl.⁵) |
| | | | C 07 D 401/00 |

Der vorliegende Recherchenbericht wurde für alle Patentansprüche erstellt.

| Recherchenort | Abschlußdatum der Recherche | Prüfer |
|---|---|---|
| WIEN | 23-08-1990 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82